# EUROPEAN PATENT APPLICATION

(11) **EP 4 151 992 A1**
(43) Date of publication of application: **22.03.2023**
(21) Application number: 21197838.2
(22) Date of filing: 20.09.2021
(51) Int. Cl.: G01N 27/12

(54) **TEMPERATURE-REGULATED CHEMI-RESISTIVE GAS SENSOR**

(71) Applicant: Infineon Technologies AG, 85579 Neubiberg (DE)
(72) Inventor: BREUER, Werner, 93161 Sinzing-Viehhausen (DE); EMMERT, Michael, 93158 Teublitz (DE); MEYER, Markus, 93161 Sinzing (DE); ROTH, Marina Alexandra, 92318 Neumarkt (DE); ZOEPFL, Alexander, 93049 Regensburg (DE); KRIVEC, Matic, 9504 Villach-Warmbad (AT)
(74) Representative: Hersina, Günter

(57) **Abstract**

The herein described innovative concept concerns a temperature-regulated chemi-resistive gas sensor (100) comprising a sensor surface (110) comprising a chemically sensitive sensor layer (101) comprising an active material (111) for adsorbing and desorbing gas molecules (133) of an analyte gas. A predetermined time-continuous periodic temperature profile (140) may be applied for periodically heating the sensor surface (110). An electrical sensor layer conductance signal (142a, 142b) may be determined and a plurality of time windows (201, ..., 205) may be applied to the sensor layer conductance signal (142a, 142b). For one or more of said plurality of time windows (201, ..., 205), discrete frequency spectrum data (144) of the sensor layer conductance signal may be obtained, and a current gas concentration of the analyte gas may be determined based on said obtained discrete frequency spectrum data (144).

## Description

The herein described innovative concept concerns a temperature-regulated chemi-resistive gas sensor and a method for operating the same. Such temperature-regulated chemi-resistive gas sensors are to be distinguished from electrochemical sensors, like metal oxide (MOx) sensors. Temperature-regulated chemi-resistive gas sensors may be heated between an adsorption temperature for allowing an adsorption of gas molecules and a desorption temperature for allowing a desorption of adsorbed gas molecules. Electrochemical sensors, like metal oxide (MOx) sensors, are instead based on chemical reactions happening at the sensor surface, where adsorbed gases will either be chemically oxidized or reduced. The herein described innovative concept concerns a temperature-regulated chemi-resistive gas sensor and a method for operating the same.

### TECHNICAL BACKGROUND

Today people may be interested in sensing gas concentrations at their current locations, e.g. at a certain current place indoors or outdoors like a room, an urban district, a vehicle, or many more. Since it is desirable to rapidly receive information about the current gas concentrations, people may want to use their every-day end user devices like mobile phones, smart phones, tablets or the like for having a gas sensor at hand. Thus, it is desirable to provide low-cost gas sensors comprising a small form factor to be included in such handy end user devices. Therefore, end users could be provided with applications for sensing environmental gases so as to receive information about current gas compositions in the environment in which the end user is currently situated.

Of course, dedicated and more sophisticated measurement devices may be available in which gas sensors may be applied. Such dedicated gas measurement devices may primarily be used in commercial applications, like professional gas sensing. Such commercially used gas sensors may be included in large stationary devices. However, both commercial and consumer applications are of interest in the field of gas sensing and, thus, are of interest in the present disclosure.

For any such gas sensing applications, different technologies of gas sensors are currently existing. For example, chemi-resistive gas sensors show a varying electrical resistivity or conductance, respectively, depending on a current gas concentration. Such chemi-resistive gas sensors may further be subdivided into two different groups, depending on their basic functional principle that causes the variation in the resistivity/conductivity.

A first group can be subsumed under the so-called electrochemical gas sensors. For example, metal oxide (MOx) gas sensors are very well established representatives of this group. Electrochemical gas sensor, and in particular MOx sensors, comprise an electrode being made of metal oxide where adsorbed gases will either be chemically oxidized or reduced. Certain metal oxides, like tin oxide (SnO₂), may vary their electrical conductance under the influence of gas. The variation of their electrical conductance may depend on the amount of measured analyte gas being present at the current measurement location. However, these sensors are subject to corrosive elements or chemical contamination and may, therefore, only have a limited operational life time.

A second group may be subsumed under the so-called temperature-regulated chemi-resistive gas sensors. An exemplary representative of this group of gas sensors may be a graphene based gas sensor. The present disclosure is concerned with this second group of gas sensors, namely with temperature-regulated chemi-resistive gas sensors.

Such temperature-regulated chemi-resistive gas sensors show a different behavior at lower temperatures compared to the above mentioned electrochemical (e.g. MOx) sensors, such as no chemical reaction of analyte gases at the sensor surface. Instead, adsorption and desorption of molecules of the analyte gas at the active sensor surface will be influenced by the temperature being applied to the active sensor surface. For example, heating the active sensor surface to a certain temperature may lead to an adsorption of analyte gas molecules at the active sensor surface. A further temperature increase of the active sensor surface may lead to a desorption of the adsorbed gas molecules at the active sensor surface. The amount of adsorbed gas molecules directly influences the electrical conductance of the active sensor surface.

Accordingly, a temperature-regulated chemi-resistive gas sensor should be heated, at least between two different temperature levels, so as to allow an adsorption and a desorption of gas molecules for sensing the analyte gas. However, only small temperature variations can be applied to these kind of gas sensors. Thus, the velocity of switching between adsorption and desorption is relatively slow. Furthermore, temperature-regulated chemi-resistive gas sensors, which are heated between at least two different temperature levels, suffer from noticeable base line drift.

Nevertheless, a temperature-regulated chemi-resistive gas sensors may often be preferable over MOx gas sensors, since temperature-regulated chemi-resistive gas sensors have a much higher sensitivity and they may even be able to sense single gas molecules.

Thus, it would be desirable to provide a temperature-regulated chemi-resistive gas sensor having a higher temporal resolution, which means that more measurement data is obtained in a certain measurement time interval compared to a conventional temperature-regulated chemi-resistive gas sensor, which leads to much faster and more precise measurement results. Furthermore, a temperature-regulated chemi-resistive gas sensor without significant base line drift, or even without any base line drift, would be desirable to further enhance the measurement results.

These goals are achieved by means of the herein disclosed temperature-regulated chemi-resistive gas sensor and the corresponding method for operating a temperature-regulated chemi-resistive gas sensor according to the independent claims. Further embodiments and advantageous aspects are suggested in the dependent claims.

A first aspect concerns a temperature-regulated chemi-resistive gas sensor comprising a MEMS device (MEMS: Micro Electro Mechanical System) comprising a heater and a sensor surface thermally coupled to the heater, the sensor surface comprising a chemically sensitive sensor layer comprising an active material for adsorbing and desorbing gas molecules of an analyte gas. The gas sensor further comprises a first circuitry configured to apply a predetermined time-continuous periodic temperature profile to the heater for periodically heating the sensor surface, wherein an electrical conductance of the sensor surface time-continuously varies in response to the applied predetermined time-continuous periodic temperature profile. The gas sensor further comprises a second circuitry configured to determine an electrical sensor layer conductance signal representing the varying electrical conductance of the sensor layer and to apply a plurality of time windows to the sensor layer conductance signal, said time windows each having a window length corresponding to a period length of a single period of the applied predetermined time-continuous periodic temperature profile. The second circuitry is further configured to obtain, for one or more of said plurality of time windows, discrete frequency spectrum data, said discrete frequency spectrum data comprising at least one of the fundamental frequency and the second harmonic of the sensor layer conductance signal. The second circuitry may further be configured to determine a current gas concentration of the analyte gas based on the obtained discrete frequency spectrum data. For example, the discrete frequency spectrum data may be obtained by applying a Discrete Fourier Transformation (DFT) or any derivation thereof.

A second aspect concerns a method for operating a temperature-regulated chemi-resistive gas sensor comprising a MEMS device having a heater and a sensor surface thermally coupled to the heater, the sensor surface comprising a chemically sensitive sensor layer comprising an active material for adsorbing and desorbing gas molecules of an analyte gas. The method comprises at least a step of applying a predetermined time-continuous periodic temperature profile to the heater for periodically heating the sensor surface, wherein an electrical conductance of the sensor surface varies in response to the applied predetermined time-continuous periodic temperature profile. The method further comprises a step of determining an electrical sensor layer conductance signal representing the varying electrical conductance of the sensor layer and applying a plurality of time windows to the sensor layer conductance signal, said time windows each having a window length corresponding to a period length of a single period of the applied predetermined time-continuous periodic temperature profile. The method further comprises a step of obtaining, for one or more of said plurality of time windows, discrete frequency spectrum data comprising at least one of the fundamental frequency and the second harmonic of the sensor layer conductance signal, and to determine a current gas concentration of the analyte gas based on the obtained discrete frequency spectrum data.

According to a third aspect, computer programs are provided, which may be stored on a computer-readable storage medium, wherein each of the computer programs is configured to implement the above-described method when being executed on a computer or signal processor, so that the above-described method is implemented by one of the computer programs.

In the following, embodiments of the present disclosure are described in more detail with reference to the figures, in which
- Fig. 1: shows a schematic overview of a gas sensing principle of a temperatureregulated chemi-resistive gas sensor according to an embodiment and a possible use case,
- Fig. 2A: shows a schematic representation of adsorption and desorption of molecules of an analyte gas on an active material to be used in a sensor layer,
- Fig. 2B: a discrete temperature profile for heating the sensor surface in discrete steps between an adsorption temperature and a desorption temperature,
- Fig. 3A: a discrete temperature profile for heating the sensor surface in discrete steps between two discrete temperatures,
- Fig. 3B: a diagram showing a sensor layer conductance signal obtainable by applying the discrete temperature profile of Figure 3A,
- Fig. 4A: a time-continuous periodic temperature profile according to an embodiment for periodically heating the sensor surface in time-continuous steps between two discrete temperatures,
- Fig.4B: a diagram showing an undistorted sensor layer conductance signal obtainable by applying the periodic time-continuous temperature profile of Figure 4A and without the presence of an analyte gas,
- Fig.4C: a diagram showing a distorted sensor layer conductance signal obtainable by applying the periodic time-continuous temperature profile of Figure 4A and in presence of an analyte gas,
- Fig. 5: a diagram showing the periodic temperature signal and the corresponding sensor layer conductance signal with and without the presence of an analyte gas,
- Fig. 6: a diagram showing a distorted sensor layer conductance signal with additional temporal windows or readout periods being triggered inside each temperature modulation period corresponding to an exemplary fivefold oversampling,
- Figs. 7A-7C: vector diagrams in a complex plane for illustrating the resulting DFT components after the sensor layer conductance signal has undergone a Fast Fourier Transformation, and an auto-phasing of complex DFT components,
- Fig. 8A: an example of data points obtained by sensing ozone as an analyte gas, wherein a sinusoidal temperature profile was applied, but without applying the herein described innovative oversampling principle,
- Fig. 8B: the same example as in Figure 8A, but with applying the herein described innovative oversampling principle,
- Figs. 9A-9E: schematic diagrams for an optional preprocessing of a sensor layer conductance signal before applying a DFT, and
- Fig. 10: a schematic block diagram for illustrating method steps of a method according to an embodiment.

Equal or equivalent elements or elements with equal or equivalent functionality are denoted in the following description by equal or equivalent reference numerals.

Method steps which are depicted by means of a block diagram and which are described with reference to said block diagram may also be executed in an order different from the depicted and/or described order. Furthermore, method steps concerning a particular feature of a device may be replaceable with said feature of said device, and the other way around.

As far as the term electrical conductivity is used herein, it is to be understood to also cover an electrical resistivity which is the reciprocal of the electrical conductivity.

As far as the term chemi-resistive is used herein, it is to be understood that this term means the same as the synonymous terms chemo-resistive and chemirestive. Thus, these three terms can be used interchangeably within the present disclosure.

As far as the term first harmonic of a signal is used herein, this corresponds to the fundamental frequency of the respective signal, i.e. the first harmonic is the same as the fundamental frequency. The second harmonic is the same as the first overtone.

The present disclosure concerns a temperature-regulated chemi-resistive gas sensor that has to be distinguished from electrochemical gas sensors, like MOx gas sensors, for example. For introductory purposes, the sensor principle and some examples of a temperature-regulated chemi-resistive gas sensor will be explained first before discussing any further details as to the present innovative concept.

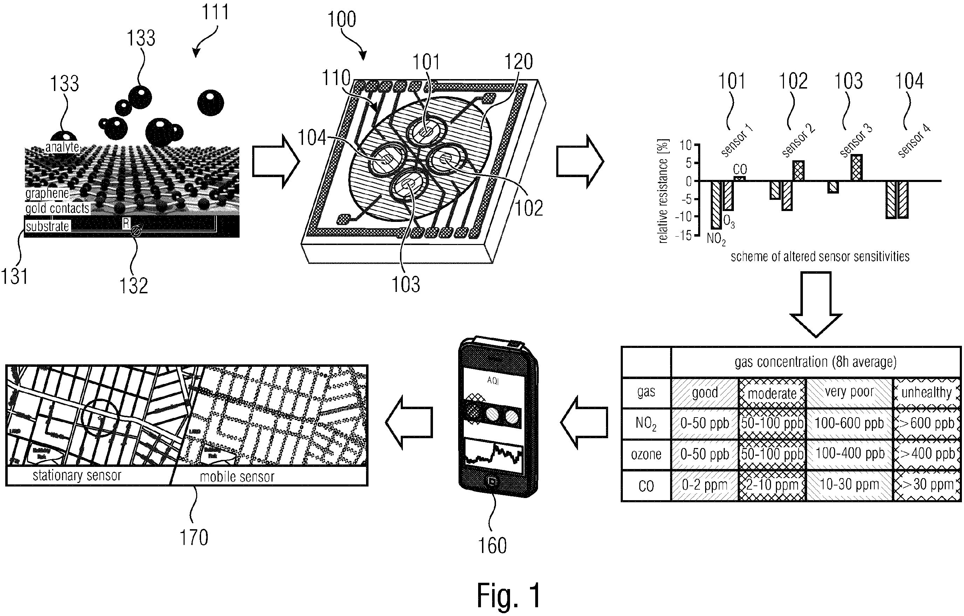

Figure 1 shows an example of a so-called Air Quality Index (AQI) monitoring that can be realized with a temperature-regulated chemo-resistive gas sensor according to the herein described innovative concept. This non-limiting example shows an AQI sensor 100 being realized as a temperature-regulated chemo-resistive gas sensor, wherein said AQI sensor 100 is configured for detecting three typical gases being suitable for determining an outdoor AQI, namely NO₂, O₃ and CO.

The AQI sensor 100 may comprise a sensor surface 110 being thermally coupled to a heater 120. The heater 120 may be constructed as a so-called MEMS (Micro Electro Mechanical System) and it may comprise an electrically regulated membrane, for example. The sensor surface 110 may comprise at least a first chemically sensitive sensor layer 101. In this non-limiting example, the sensor surface 110 may additionally comprise a second, third and a fourth chemically sensitive sensor layer 102, 103, 104.

Each of the chemically sensitive sensor layers 101, 102, 103, 104 may comprise an active material for adsorbing and desorbing molecules 133 of an analyte gas, or analyte gas mixture.

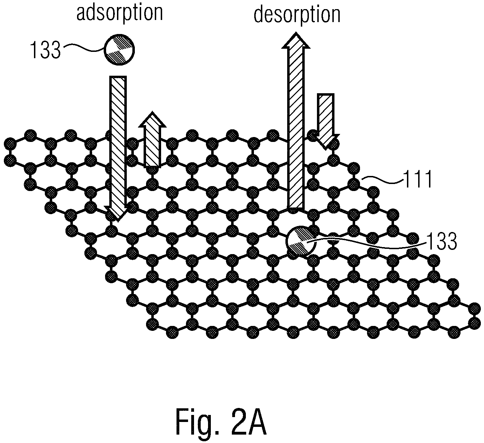

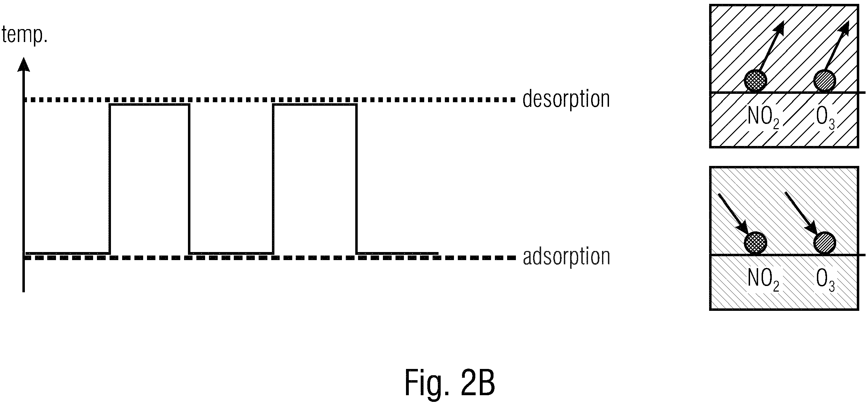

The top left picture of Figure 1 shows an enlarged detail of an active material 111 of one of the chemically sensitive sensor layers 101, 102, 103, 104, assume sensor layer 101. The active material 111 may be provided as a layer being arranged, e.g. deposited, printed or dispensed, on an interdigital structure being provided on a substrate 131. The active material 111, and thus the sensor layer 101, may comprise an electrical idle resistivity, e.g. at a reference temperature and/or with the presence of a reference gas (e.g. synthetic air). If the active material 111 is heated by means of the heater 120, up to a first temperature range, then the active material 111 may begin to adsorb molecules 133 of an analyte gas (c.f. Figures 2A and 2B). Depending on the number of molecules 133 being adsorbed, a charge transfer appears which leads to an alteration of the electrical resistivity 132 of the sensor layer 101 (compared to its idle resistivity). This increase or decrease in electrical resistivity 132 can be measured.

If the temperature is further increased, up to a second higher temperature range, the active material 111 of the chemically sensitive sensor layer 101 may be recovered, i.e. adsorbed molecules 133 will be desorbed again (c.f. Figures 2A and 2B). In this case, the electrical resistivity 132 will alter again.

For example, if the resistivity of the active material 111 may comprise an NTC behavior (NTC: Negative Temperature Coefficient), then its electrical conductance will rise with increasing temperature, and it will fall with decreasing temperature. Accordingly, if the temperature is raised from the first temperature range up to the higher second temperature range, the electrical conductance of the active material 111 will also increase accordingly. In turn, if the temperature is reduced, the electrical conductance of the active material 111 will decrease.

To be more precise not each and every molecule 133 will be adsorbed at the lower temperature, and in turn, not each and every adsorbed molecule 133 will be desorbed again at the higher temperature. In reality, the equilibrium of adsorption and desorption shifts, i.e. in the higher temperature range more molecules 133 will be desorbed compared to the lower temperature range, and the other way around.

The active material 111 of the at least one chemically sensitive sensor layer 101 may comprise a conducting or a semi-conducting material having an electrical conductivity that is variable based on the type and/or number of gas molecules that are adsorbed at the surface of said material.

The active material 111 may comprise at least one of
- a graphene-based material,
- modified graphene,
- a mono-crystalline graphene monolayer, or
- a 2D material.

Additionally or alternatively to graphene-based materials, the active material 111 may comprise thin layers comprising metal, metal oxides, metal sulfides or semiconductors. The structure of these layers may be poly crystalline or amorphous. Also real 2D-materials may be used as the active material 111, such as mono-crystalline graphene monolayers or topological insulators.

2D-materials refer to crystalline solids consisting of a single layer of atoms. 2D-materials may therefore also be referred to as single-layer materials. They may be derived from single elements, or from compounds of two or more elements. 2D materials may generally be categorized as either 2D allotropes of various elements or as compounds (consisting of two or more covalently bonding elements).

As a non-limiting example, the active material 111 may, for instance, comprise or be made from graphene, and in particular modified graphene, which will also be referred to as functional or functionalized graphene. That is, the chemical and/or electrical properties of functional graphene will be modified compared to pure graphene. For example, the active material 111 may comprise graphene flakes which are mixed with functional nanoparticles or salts, wherein the latter influence the behavior of the otherwise pure graphene flakes. Accordingly, the sensitivity of the modified graphene may be adjusted such that the modified or functionalized graphene may become sensitive to certain types and/or concentrations of gas.

For example, the different chemically sensitive sensor layers 101, 102, 103, 104 may each comprise a differently modified active material, e.g. a differently functionalized graphene layer, which makes the sensor layers 101, 102, 103, 104 sensitive to different types of gases and/or different concentrations of gases. That means, even though an identical concentration of an analyte gas (mixture) is present at the sensor surface 110, each sensor layer 101, 102, 103, 104 may determine a different type of gas and a different concentration of gas due to their differently modified active materials. A corresponding example is shown at the top right picture of Figure 1.

By suitably interpreting the measured results, the actual gas concentration may be evaluated and may be displayed to a user, e.g. by means of a smart phone device 160 or the like. If a plurality of such mobile AQI sensors are connected with each other, it may become possible to create a map 170 showing the actual Air Quality Index in different regions, e.g. in different streets.

Sensing the kind of air pollution and estimation of the gas concentrations with a conventional gas sensor by performing a simple static resistivity measurement seems to be easy. However, in practical applications, such a conventional gas sensing method is affected by several problems. E.g. base line drift, noise, cross sensitive, aging (poisoning), and the like.

There are some possibilities to improve the functionality a little bit. As mentioned before, the chemically sensitive sensor layers 101, 102, 103, 104 may be alternatingly heated to a first temperature (e.g. an adsorption temperature) and to a higher second temperature (e.g. a desorption temperature). That is, the operating temperature of the temperature-regulated chemo-resistive gas sensor 100 is modulated with a certain modulating scheme.

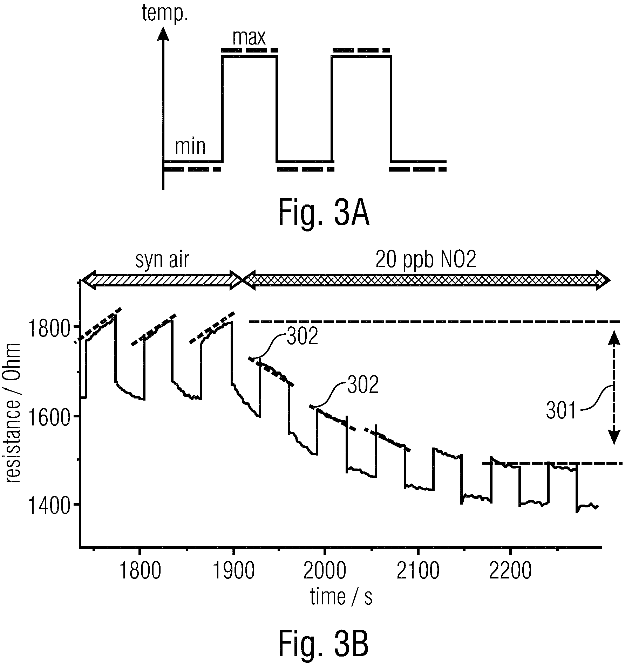

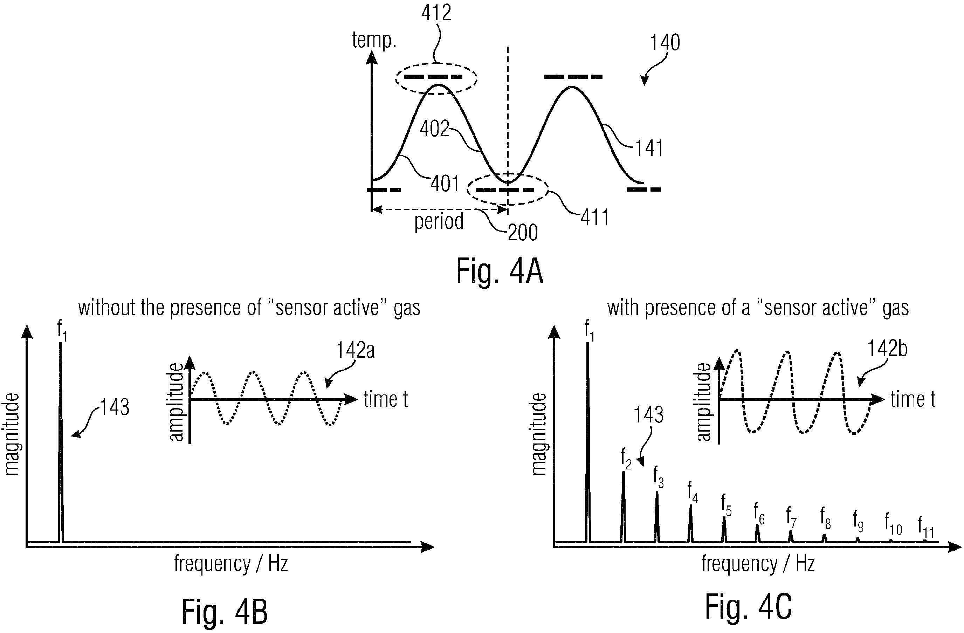

Figures 3A and 4A show two examples of modulating schemes. According to Figure 3A, a binary pulsed modulating scheme is applied. That is, the temperature is discretely modulated by means of binary pulses, wherein the operating temperature is switched between two discrete temperatures, namely the lower first temperature for sensing (adsorption) and the higher second temperature for recovering (desorption) the sensor 100.

Figure 4A shows a dynamic sensing method comprising a periodic temperature signal, wherein the temperature is continuously altered between the first lower temperature (range) and the second higher temperature (range). The herein described innovative concept makes use of this dynamic sensing method comprising the periodic temperature signal, details and examples of which will be described somewhat later in this text.

Figures 3B, 4B and 4C deem to explain the differences in the signal analysis of the measured conductance signals of the sensor. In particular, Figures 3B, 4B and 4C show the varying signal path of the alternating conductance of an exemplary one of the sensor layers 101, 102, 103, 104. As mentioned above, the electrical conductance of the sensor layers 101, 102, 103, 104 varies with the varying temperature applied by the heater 120.

In this regard, Figures 4A and 4B show the signal path of the applied temperature, i.e. the temperature signal, while Figures 3B, 4B and 4C show the signal path of the correspondingly varying electrical resistivity of the sensor layers 101, 102, 103, 104, i.e. the sensor layer resistivity signals. Of course, in case the electrical conductance was measured instead of the resistivity, the signal paths would accordingly symbolize the sensor layer conductance signals.

As can be seen in Figure 3B, the sensor layer resistivity signal follows, in discrete steps, the discrete temperature signal (Figure 3A). If synthetic air is present at the sensor surface 110, the median of the electrical resistivity signal stays at the same level. However, if an analyte gas is applied (e.g. 20 ppb of NO₂), the median of the electrical resistivity signal decreases, i.e. the conductivity increases. Arrow 301 symbolizes the relative resistivity, i.e. the decrease of the resistivity signal, between synthetic air and the analyte gas. Furthermore, for each discrete resistivity signal portion, the corresponding derivative can be determined.

Both the relative resistivity and the derivative are individual for each gas and each active material. However, there is a drawback in that no dynamic information may be derived from these discrete signals. For example, different types of gases may show a different behavior as to their adsorption and desorption. In other words, the adsorption and/or desorption mechanism may start at different temperatures for different gases. However, this information is not derivable from discrete signals as shown in Figures 3A and 3B.

A further development is shown in Figure 4A, where a dynamic measurement mode comprising a periodic temperature signal 141 is shown. As mentioned above, a periodic temperature profile 140 is applied, i.e. the temperature is modulated dynamically, which means that the temperature is continuously altered between the first lower temperature (range) and the second higher temperature (range). The applied predetermined periodic time-continuous temperature profile 140 may be represented by a periodic time-continuous temperature profile signal 141. In some non-limiting examples, the periodic temperature profile signal 141 may be sinusoidal.

According to an embodiment, the applied predetermined time-continuous periodic temperature profile signal 141 has a time-continuous temperature increase 401 and a time-continuous temperature decrease 402 during the period length of one single period 200. This is a decisive difference over the previously described discrete temperature profile (Figure 3A) wherein the temperature is switched between two discrete temperature values.

In order to apply such a periodic time-continuous temperature profile 140 as shown in=Figure 4A, the temperature-regulated chemo-resistive gas sensor 100 may comprise a precise temperature regulation with feedback control.

Thus, according to an embodiment, the first circuitry may comprise a feedback-controlled temperature regulation for controlling the applied predetermined time-continuous periodic temperature profile 140 such that, during the period length of one single period 200 of the periodic temperature profile signal 141, the predetermined time-continuous periodic temperature profile signal 141 passes a first target temperature range 411 at which the sensor layer 101 adsorbs gas molecules 133 of the analyte gas and passes a second target temperature range 412 at which the sensor layer 101 desorbs adsorbed gas molecules 133 of the analyte gas. That is, the sensor layer 101 may desorb at least a part of the entire adsorbed gas molecules 133.

Assumed that the periodic time-continuous temperature profile 140 of Figure 4A was applied, Figures 4B and 4C show the corresponding sensor layer conductance signal 142a, 142b, exemplarily for one of the sensor layers 101, 102, 103, 104. As can be seen, the sensor layer conductance signal 142a, 142b (Figures 4B, 4C) follows the periodic temperature signal 141 (Figure 4A). Thus, the sensor layer conductance signal 142a, 142b is also periodic.

Without the presence of "sensor active" gas molecules (Figure 4B) the periodic sensor layer conductance signal 142a, 142b follows the periodic (e.g. sinusoidal) temperature signal 141 (Figure 4A). Since temperature dependency and timescale of adsorption and desorption of gas molecules may be different for different gas molecules 133, the presence of different gas molecules 133 may cause a specific distortion of the periodic sensor layer conductance. As can be seen in Figure 4C, the presence of an analyte gas causes a specific distortion of the periodic sensor layer conductance signal 142b compared to an undistorted periodic sensor layer conductance signal 142a.

The periodic sensor layer conductance signal 142a, 142b may be subjected to a Discrete Fourier Transformation (DFT). As a result, the frequency components which contribute to the distortion of the periodic sensor layer conductance signal 142a, 142b may be determined. Thus, a discrete frequency spectrum 143 can be obtained by applying a DFT, as shown in Figure 4C. In this obtained DFT-spectrum 143, the distortion of sinusoidal signals is reflected by the harmonic amplitudes. Since the distortion depends on the type and concentration of the analyte gas, each analyte gas may have its own fingerprint that is detectable when applying a DFT analysis.

One of the advantages of this method, which uses a periodic temperature profile 140, is that the dynamic behavior (e.g. start temperatures of adsorption/desorption) of the gases as well as the individual fingerprints of different gas types may be determined by a preprocessing of the dynamic data with DFT. That is, without the presence of any analyte gas ("sensor active" gas), a periodic temperature profile signal 141 is reflected by a periodic undistorted sensor layer conductivity signal 142a. In turn, the presence of an analyte gas ("sensor active" gas), leads to a distorted periodic sensor layer conductance signal 142b. Different types and/or concentration of gases lead to different distortions.

However, one problem related with temperature-regulated chemo-resistive gas sensors 100 in general is that only small temperature amplitudes can be applied. This means that the velocity of adsorption/desorption is quite slow. Depending on the gas concentration, a distortion of the periodic sensor layer conductance signal 142b can be quite small (just a slight phase shift of first harmonics in DFT signal). For high gas sensitivities long periods of the applied temperature profile 140 (in the range of some minutes) are needed. Analyzing the distortion of each period gives a sensing rate of just one data point per temperature period, i.e. only one data point within some minutes.

Stated in other words, conventional temperature-regulated chemo-resistive gas sensors 100 are very slow. They need several minutes or even hours to output a valid result of the measured gas types and/or gas concentrations.

The temperature-regulated chemo-resistive gas sensor 100 according to the herein described innovative principle provides for considerably faster sensor responses. For this purpose, the temperature-regulated chemo-resistive gas sensor 100 according to an embodiment is comparable to the one described above with reference to Figure 1, top middle picture. The temperature-regulated chemo-resistive gas sensor 100 comprises a MEMS device (MEMS: Micro Electro Mechanical System) comprising a heater 120 and a sensor surface 110 thermally coupled to the heater 120. The sensor surface 110 comprises at least one chemically sensitive sensor layer 101 comprising an active material 111 for adsorbing and desorbing gas molecules 133 of an analyte gas.

The temperature-regulated chemo-resistive gas sensor 100 may further comprise a first circuitry (not shown) configured to generate a predetermined time-continuous periodic temperature profile 140, such as the one shown in Figure 4A, represented by means of the corresponding temperature profile signal 141, and to apply this time continuous temperature profile 140 to the heater 120 for periodically heating the sensor surface 110. As mentioned above, an electrical conductance of the sensor layer 101 varies in response to the applied predetermined time-continuous periodic temperature profile 140.

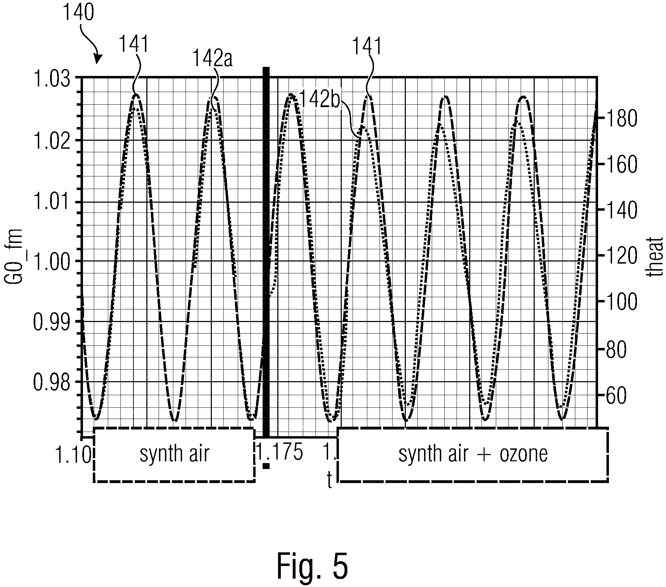

The temperature-regulated chemo-resistive gas sensor 100 may further comprise a second circuitry (not shown) configured to determine an electrical sensor layer conductance signal 142 (c.f. Figures 4A, 4B) representing the varying electrical conductance of the sensor layer 101. For further explanation of the herein described innovative principle, reference shall now be made to Figure 5.

Figure 5 shows an example plot of a temperature profile signal 141 representing the applied periodic and time-continuous temperature profile 140. Figure 5 also shows the sensor layer conductance signal 142a, 142b. The left part of the plot shows the undistorted sensor layer conductance signal 142a that appears if a reference gas, e.g. synthetic air, is present at the sensor surface 110. The right part of the plot shows the distorted sensor layer conductance signal 142b that appears if an analyte gas, e.g. ozone, is present at the sensor surface 110.

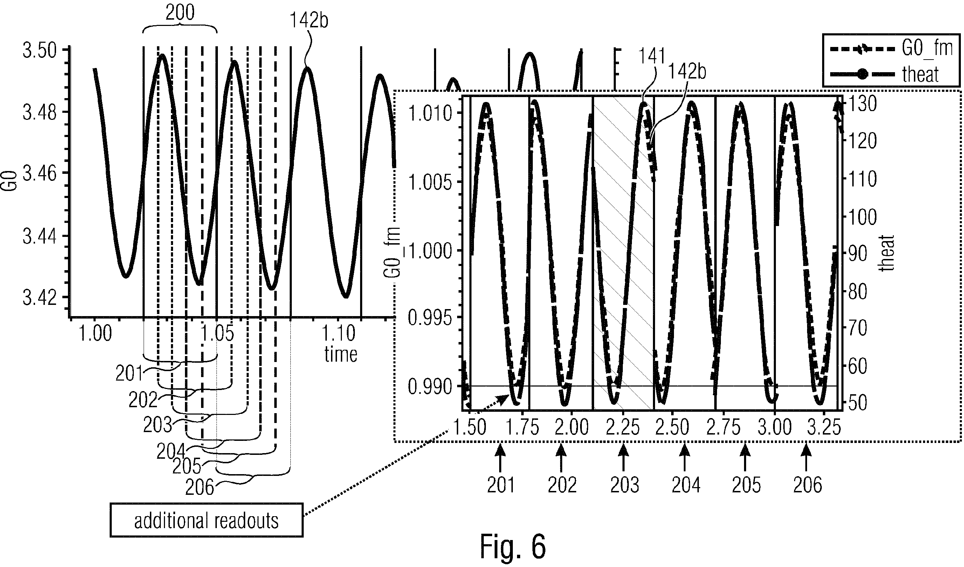

Figure 6 shows an important feature of the present innovative concept. The second circuitry is configured to apply a plurality of time windows 201, ..., 205 to the sensor layer conductance signal 142b. Each one of the time windows 201, ..., 205 has a window length that exactly corresponds to a period length of a single period 200 of the periodic temperature profile signal 141. Since the periodic sensor layer conductance signal 142b follows the periodic temperature profile signal 141, the period length of the periodic sensor layer conductance signal 142b may be the same as the period length of the periodic temperature profile signal 141. Accordingly, each one of the time windows 201, ..., 205 may have a window length corresponding to a period length of a single period 200 of the periodic sensor layer conductance signal 142b.

The additional time windows 201, ..., 205 may be started during exactly one period 200, i.e. between the beginning and the end of exactly one period 200. Thus, according to an embodiment, the second circuitry may be configured to start the plurality of time windows 201, ..., 205 during a period length of one single period 200 of the predetermined time-continuous periodic temperature profile signal 141.

This results in additional readouts comparable to an oversampling. As shown in the right part of Figure 6, each time window 201, ..., 205 generates a corresponding readout covering exactly one period of the periodic temperature profile signal 141 and the resulting (distorted) sensor layer conductance signal 142b. However, each additional readout starts at a different phase angle, which is clear since each of the additional time windows 201, ..., 205 is started within exactly one period length 200. Accordingly, one can say that each additional time window 201, ..., 205 creates an additional readout that shows a snippet of the (distorted) sensor layer conductance signal 142b, and optionally also of the periodic temperature profile signal 141, wherein each snippet covers exactly one period length. However, as mentioned above, the phase angle differs in each snippet.

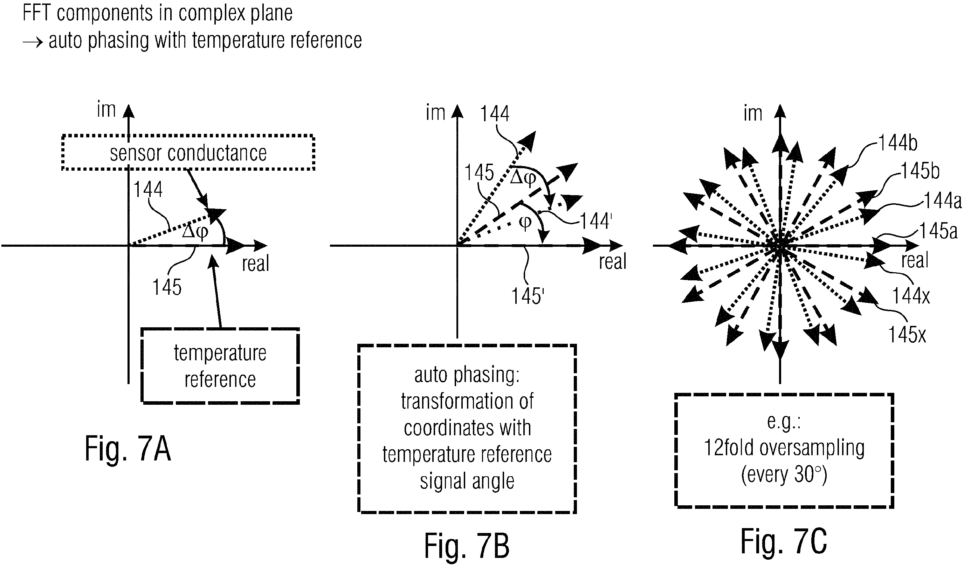

Figures 7A, 7B and 7C then show a further important feature of the present innovative concept. Discrete frequency spectrum data of the sensor layer conductance signal 142b may be obtained for each of said one or more time windows 201, ..., 205. For doing so, the second circuitry may be configured to apply a Discrete-Fourier-Transformation (DFT) to one or more of said plurality of time windows 201, ..., 205, and preferably to each one of the plurality of time windows 201, ..., 205. Thereby, discrete frequency spectrum data of the DFT-transformed (distorted) sensor layer conductance signal 142b can be obtained for each of said one or more time windows 201, ..., 205. Said discrete frequency spectrum data represents a current gas concentration of the analyte gas.

In some embodiments, the second circuitry may be configured to apply at least one of a Discrete Fourier Transformation (DFT), a Fast Fourier Transformation (FFT), a Short-Time Fourier Transformation (STFT) or a Goertzel-Algorithm to said one or more time windows 201, ..., 205 for obtaining the discrete frequency spectrum data of the sensor layer conductance signal. It is to be noted that an FFT, a STFT and the Goertzel-Algorithm may be considered as particular forms of a Discrete Fourier Transform (DFT). Thus, DFT may be used as the generic term covering an FFT, a STFT and the Goertzel-Algorithm. Accordingly, as far as the term DFT is used herein, it also covers a FFT, a STFT and a Goertzel-Algorithm.

The discrete frequency spectrum data, that is obtained after applying one of the DFT, STFT, FFT or the Goertzel-Algorithm, may be represented by a discrete frequency spectrum 143, as discussed above with reference to Figure 4C. In a different representation, the obtained discrete frequency spectrum data may be represented by vectors 144, 145 in the complex plane, said vectors 144, 145 having a real part and an imaginary part. For example, the periodic (distorted) sensor layer conductance signal 142b may be subjected to a DFT, which results in a DFT-transformed sensor layer conductance signal 144. Additionally or alternatively, the periodic temperature profile signal 141 may optionally be subjected to a DFT, which results in a DFT-transformed temperature profile signal, represented by vector 145.

Figure 7A shows the result of applying a DFT to exactly one time window, wherein vector 144 represents the DFT components of the DFT-transformed sensor layer conductance signal, and vector 145 represents the DFT components of the DFT-transformed temperature profile signal. Since the phase angle ϕ of the DFT-transformed temperature profile signal 145 is zero (ϕ = 0°), the first time window 201 of Figure 6 was used here.

As can be seen in Figure 7A, a differential phase angle Δϕ can be determined between the two vectors 144, 145, i.e. between the DFT-transformed sensor layer conductance signal 144 and the DFT-transformed temperature profile signal 145. This differential phase angle Δϕ represents the above discussed distortion of the periodic sensor layer conductance signal 142b relative to the periodic temperature profile signal 141 acquired during the period length of exactly one time window 201. As mentioned above, this distortion, i.e. the differential phase angle Δϕ in the complex plane, is specific for each type and/or concentration of analyte gas. Thus, the differential phase angle Δϕ represents the above mentioned fingerprint being specific for each type and/or concentration of analyte gas. Accordingly, the differential phase angle Δϕ indicates the sensed type and/or concentration of the analyte gas. Thus, the differential phase angle Δϕ may also be referred to as a gas-specific phase angle.

Thus, according to an embodiment, the second circuitry may be configured to determine, for at least one of the applied plurality of time windows 201, ..., 205, a gas-specific phase angle Δϕ between the DFT-transformed sensor layer conductance signal, i.e. vector 144, belonging to said at least one time window 201, ..., 205 and the temperature profile, represented by vector 145, applied during the window length of said at least one time window 201, ..., 205. The second circuitry may further be configured to determine the current gas concentration of the analyte gas based on said gas-specific phase angle Δϕ.

In this non-limiting example as depicted in Figures 7A, 7B and 7C the periodic temperature profile signal 141 was subjected to an FFT analysis, resulting in the above mentioned FFT-transformed temperature profile signal being represented by vector 145. However, since FFT may be a subtype of DFT, any kind of DFT may be applied as well. Thus, according to an embodiment, the second circuitry may be configured to apply a DFT, e.g. a Fast Fourier Transformation (FFT), to the temperature profile 140 that is applied during the window length of said at least one time window 201, ..., 205 in order to obtain a DFT-transformed (e.g. FFT-transformed) temperature profile signal, represented by vector 145, for said at least one time window 201, ..., 205. The second circuitry may further be configured to determine, for said at least one time window 201, ..., 205, the gas-specific phase angle Δϕ between the DFT-transformed (e.g. FFT-transformed) sensor conductance signal, represented by vector 144, and the DFT-transformed (e.g. FFT-transformed) temperature profile signal, represented by vector 145.

However, this is only an optional step of the herein described innovative concept. As will be explained further below, the temperature profile signal 141 will serve as a reference for determining the gas-specific differential phase angle Δϕ relative to the DFT-transformed sensor layer conductance signal represented by vector 144. However, since the signal path of the periodic temperature profile signal 141 is known in advance, the second circuitry does not have to necessarily apply a DFT to the periodic temperature profile signal 141. Instead the periodic temperature profile signal 141 can be used directly as a reference to determine the gas-specific phase angle Δϕ.

Figure 7B shows a further example of the vectors 144, 145. In this case, vector 145 has a phase angle ϕ that is different from zero. Accordingly, one of the other time windows 202, ..., 205 was applied here, for example the second time window 202. However, vector 145 may be turned back to ϕ = 0°, as symbolized by vector 145' shown in dashed lines. The same holds for vector 144 that can be turned back to vector 144'. Turning back the vectors 144, 145 to their initial position of ϕ = 0° results in the same vector diagram as shown in Figure 7A. This may be referred to herein as auto-phasing.

However, as mentioned further above, if only one measurement is conducted within one period, the sensor response time is very slow. Thus, Figure 7C shows an example wherein twelve additional time windows were applied during one period, in the same way as previously discussed with reference to Figure 6. Accordingly, twelve measurements were conducted during one period. As can be seen in Figure 7C, for each applied time window, a pair of vectors is obtained. For example, during the first time window, a first pair of vectors 144a, 145a is obtained. During the second time window, a second pair of vectors 144b, 145b is obtained, and so on.

In this non-limiting example shown in Figure 7C, the differential phase angle Δϕ stays the same between each pair of vectors 144a, 145a, ..., 144x, 145x. This means, that the concentration of the analyte gas did not change during appliance of these exemplary twelve time windows. However, if the concentration of the analyte gas would change during one of the time windows, then the differential phase angle Δϕ would also change (increase or decrease) between the respective pair of vectors 144, 145 belonging to said time window.

Summarizing, the time windows 201, ..., 205, and preferably each one of the time windows 201, ..., 205, will be subjected to a DFT (e.g. an FFT). At least the distorted sensor layer conductance signal 142b contained inside said time windows 201, ..., 205 will be transformed by the DFT. Optionally, the temperature profile signal 141 may also be transformed by the DFT. The result will be a vector 144, 145 in the complex plane, each vector representing the phase and magnitude of the DFT-transformed signal 142b, 141. The temperature vector 145 serves as a reference for the conductance vector 144, in order to determine a differential phase angle Δϕ between the two vectors 144, 145. Depending on the type and/or concentration of the analyte gas, the conductance vector 144 deviates (by the differential phase angle Δϕ) from the temperature vector 145. Thus, the differential phase angle Δϕ represents the deviation of the sensor layer conductance signal 142b towards the periodic temperature profile signal 141. In other words, the differential phase angle Δϕ represents the distortion of the sensor layer conductance signal 142b. Each gas may have its own fingerprint that can be read from the vector diagram as shown in Figures 7A, 7B and 7C.

In general, the discrete frequency spectrum data 143 (c.f. Figure 4C) of the DFT-transformed sensor layer conductance signal correspond to harmonics of the frequency of the applied temperature profile 140. The embodiments that were discussed above with reference to Figures 7A-7C only show the first harmonic, which means that the gas-specific phase angle Δϕ was determined based on processing the first harmonic only. However, the second, third, fourth, and so on, harmonics may be used in the innovative concept. Accordingly, the second circuitry may be configured to determine the current gas concentration of the analyte gas based on at least the first harmonic, or based on the first harmonic only.

Furthermore, the present innovative concept applies exactly one time window over exactly one period length of the temperature profile signal 141 and/or the sensor layer conductance signal 142b, respectively. Applying a DFT to such a time window has the advantage that only discrete values will be obtained in the frequency spectrum, i.e. only the base frequency and its harmonics.

Furthermore, this concept considerably saves computing time since only exactly one period of the signal has to be transformed by the DFT, instead of applying a DFT to a larger time window over several periods, or even over the entire signal duration.

Furthermore, the present innovative concept applies a plurality of consecutive time windows 201, ..., 205, wherein each time window covers exactly one period of the applied temperature profile signal 141. Briefly returning to Figure 6 (left), one can see that each of the consecutive time windows 201, ..., 205 is started within, i.e. during, one and the same single period 200 of the temperature profile signal 141. Accordingly, a plurality of additional readouts (corresponding to the number of additionally applied time windows 201, ..., 205) will be obtained. Again, each readout covers exactly one period leading to the above discussed advantages.

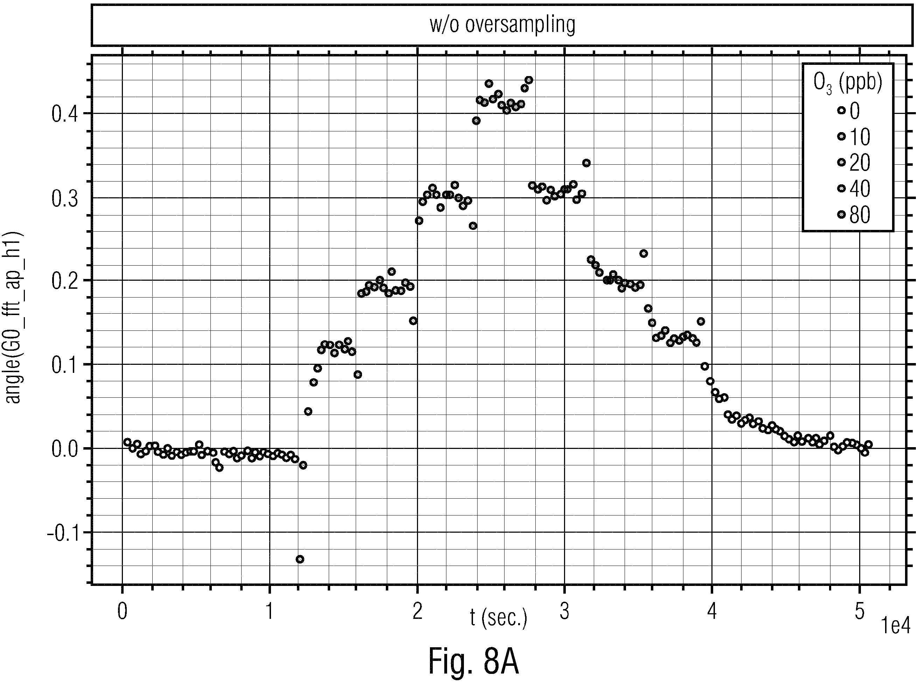

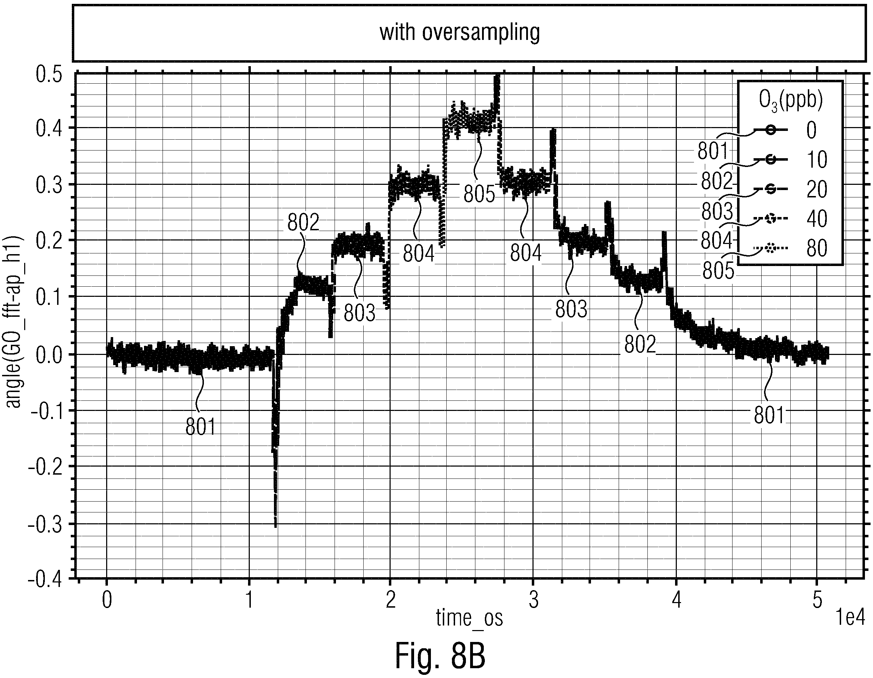

The appliance of the plurality of additional time windows 201, ..., 205 may be comparable to an oversampling. For example, the present innovative concept may be applied with a sample rate of 1/s, which means that about 300 samples (measurement results) can be obtained over one period of the temperature profile signal 141. This shall be discussed in the following with reference to Figures 8A and 8B.

Figure 8A shows a plot of obtained measurement results, also referred to as samples or readouts, without the herein described innovative oversampling technique. Thus, conventional temperature-regulated chemo-resistive gas sensors only get exactly one sample per period. As mentioned above, temperature-regulated chemo-resistive gas sensors in general have a rather slow sensor response since the time duration (period) of heating the sensor between the adsorption temperature and the desorption temperature may take up to several minutes. Accordingly, conventional gas sensors may gather only exactly one sample during this time duration of several minutes. This is shown in Figure 8A.

Figure 8B shows a plot of measurement results, i.e. samples or readouts, that may be obtained with a temperature-regulated chemo-resistive gas sensor according to the herein described innovative principle. As mentioned above, a plurality of additional time windows 201, ..., 205 will be started during exactly one period length 200. Accordingly, a plurality of additional readouts may be created in exactly one period and, thus, a lot more samples will be generated during one period between adsorption temperature and desorption temperature, compared to Figure 8A. This becomes evident when looking at the much denser arrangement of samples in Figure 8B.

Still with reference to Figure 8B, the gas concentration of the analyte gas can be easily detected. In this example, ozone (O₃) is the analyte gas. Reference numeral 801 indicates a gas concentration of 0 ppb (parts per billion) of O₃, reference numeral 802 indicates a gas concentration of 10 ppb of O₃, reference numeral 803 indicates a gas concentration of 20 ppb of O₃, reference numeral 804 indicates a gas concentration of 40 ppb of O₃, and reference numeral 805 indicates a gas concentration of 80 ppb of O₃.

Again, since the present innovative concept provides for a lot more samples (Figure 8B), the signal response of the sensor becomes considerably more accurate and precise compared to gas sensors that do not make use of the present innovative concept (Figure 8A). Accordingly, a currently present gas concentration can be detected way faster and, thus, can be displayed to the user considerably quicker. For example, the plot in Figure 8A delivers one sample every five minutes, while the plot of Figure 8B delivers one sample every five seconds.

Furthermore, if the derivative of the large-signal (considering the depicted plot as one large graph with a maximum at 805) is considered, then it may also be obtained whether the concentration of the analyte gas is increasing (left half of the plot) or decreasing (right half of the plot).

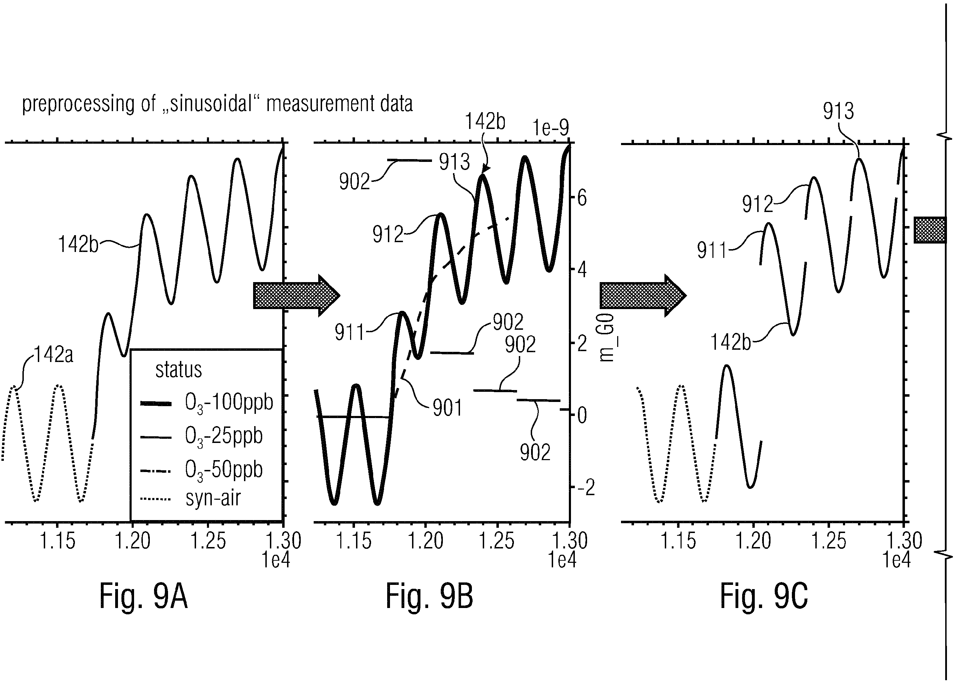

Figures 9A to 9E show some detailed views of a graph of the sensor layer conductance signal 142b that becomes distorted as an analyte gas (O₃ @ 25 ppb) is applied to the sensor surface 110. According to an embodiment, a preprocessing of the sensor layer conductance signal 142b may be executed prior to applying the DFT. In the non-limiting example of Figures 9A to 9E, a sinusoidal signal path of the sensor layer conductance signal 142b is shown. However, any other kind of a periodic signal can be used.

Figure 9A shows the undistorted sensor layer conductance signal 142a if no analyte gas is present, e.g. only synthetic air may be present that serves as a reference gas. Figure 9A also shows the distorted sensor layer conductance signal 142b that becomes distorted in case an analyte gas is present, in this case ozone O₃ with a concentration of 25 ppb.

Figure 9B shows that the mean slope 902 of the signal 142b is determined for each pulse 911, 912, 913. Each pulse 911, 912, 913 corresponds to exactly one period and, thus, to exactly one window length. For illustrative purpose, the actual slope 901 of the signal 142b is also depicted in Figure 9B.

As shown in Figure 9C, the determined mean slope 902 may be used for flattening each of the pulses, as exemplarily shown for pulses 911, 912, 913.

Thus, according to an embodiment, the second circuitry may be configured to, prior to applying the Discrete Fourier Transformation, determine, for at least one of the applied plurality of time windows 201, ..., 205, a mean slope 902 of the sensor layer conductance signal 142b contained inside said at least one time window 210, ..., 205, and to use the determined mean slope 902 for flattening the sensor layer conductance signal 142b contained inside said at least one time window 201, ..., 205.

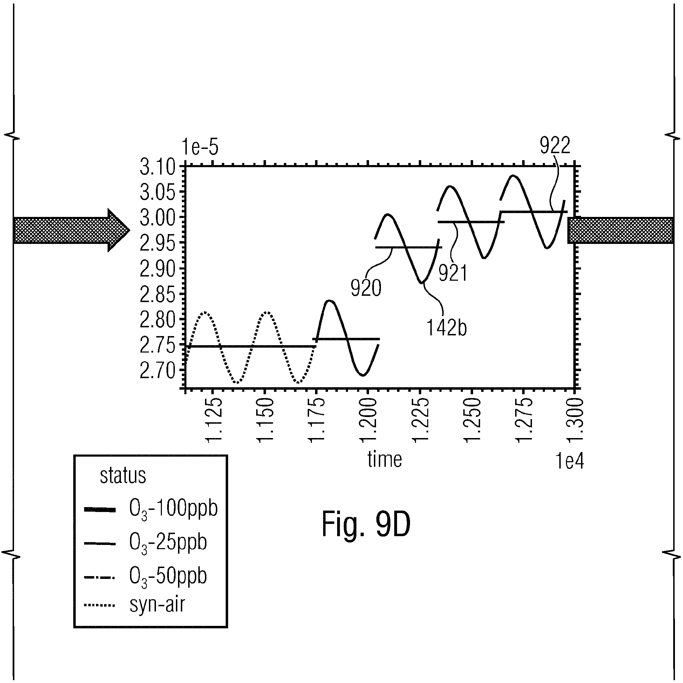

As shown in Figure 9D, a mean value of each pulse 911, 912, 913 of the sensor layer conductance signal 142b may be determined, as exemplarily depicted for the mean values 920, 921, 922.

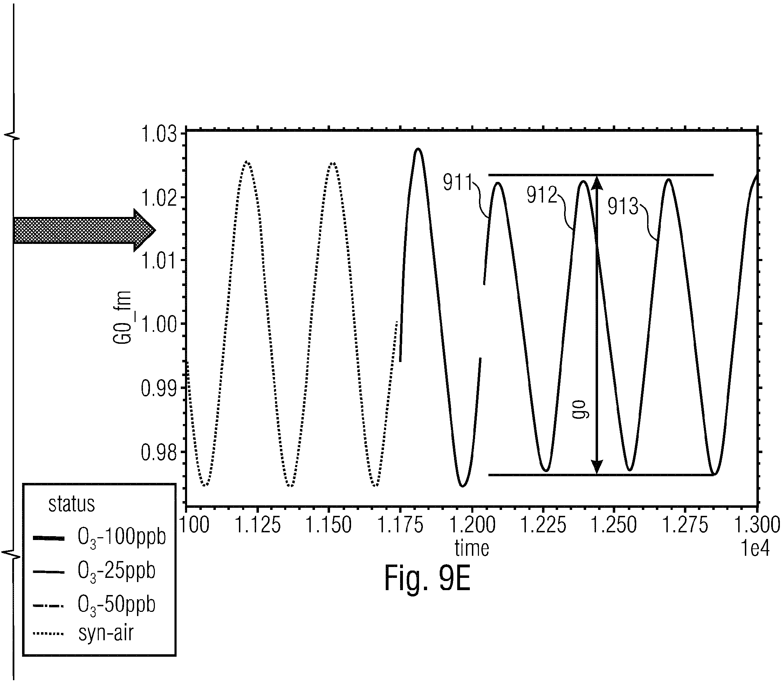

As shown in Figure 9E, the flattened pulses 911, 921, 913 can then be normalized with a normalization factor, e.g. a normalized conductance value Go, as symbolized with the double-head arrow in Figure 9E.

Thus, according to an embodiment, the second circuitry may be configured to, prior to applying the Discrete Fourier Transformation, determine, for at least one of the applied plurality of time windows 201, ..., 205, a normalization factor, and to apply said normalization factor to the sensor layer conductance signal 142b contained inside said at least one time window 201, ..., 205.

Again, the term "time window" here corresponds to the term "pulse" with reference to Figures 9A to 9E. Accordingly, a time window 201, ..., 205 and a pulse 911, 912, 913 may be used interchangeably.

After flattening and normalizing, the flattened and normalized pulses 911, 912, 913 of the distorted sensor layer conductance signal 142b can then be subjected to the DFT, as discussed above.

In the above discussed embodiments, the innovative temperature-regulated chemo-resistive gas sensor 100 was exemplarily described with reference to one sensor layer 101 (c.f. Figure 1, top middle). A further embodiment may comprise a second chemically sensitive sensor layer 102, as mentioned above with reference to Figure 1.

This second chemically sensitive sensor layer 102 may have a sensitivity to a type and/or concentration of the analyte gas, which sensitivity is different from the previously discussed at least one chemically sensitive sensor layer 101, which will be referred to in the following as a first sensor layer 101.

Said different sensitivity may be provided in that the active material 111 of the first sensor layer 101 may be different or differently modified compared to the active material 111 of the second sensor layer 102.

However, everything that have been mentioned herein with respect to the first sensor layer 101 exactly holds for the second sensor layer 102. In particular, the second sensor layer 102 may also be subjected to the periodic and time-continuous temperature profile 140 resulting in a periodic sensor layer conductance signal 142a, 142b. Based on the type and/or concentration of gas, the sensor layer conductance signal 142a, 142b of the second sensor layer 102 may also be distorted somehow. However, since the sensitivities of the two sensor layers 101, 102 may differ, as described above, the distortion of the sensor layer conductance signal of the first and second sensor layers 101, 102 may also differ accordingly even though an identical concentration of the analyte gas may be present at the sensor surface 110, i.e. at both sensor layers 101, 102.

Summarizing, for separating signals of different types and/or concentration of gases, a plurality of chemically sensitive sensor layers 101, 102, 103, 104 may be provided, wherein each sensor layer 101, ..., 104 may comprise a different or a differently modified active material. These different sensor layers 101, ..., 104 provide differently distorted sensor layer conductance signals 142b for one and the same type and/or concentration of gas (with respect to both the absolute conductance values 142b and the DFT-transformed components 144). An algorithm, e.g. implemented in the second circuitry, may summarize the obtained sensor layer data of each sensor layer 101, ..., 104 and determine the type and/or concentration of analyte gas. The algorithm may use an artificial intelligence, e.g. a neural network.

Thus, according to an embodiment, the sensor surface 110 may comprise at least a second chemically sensitive sensor layer 102 comprising an active material 111 for adsorbing and desorbing gas molecules of the analyte gas, wherein said second chemically sensitive sensor layer 102 may comprise a differently modified active material 111 than the at least one (i.e. first) chemically sensitive sensor layer 101.

The second circuitry may be configured to determine a second electrical sensor layer conductance signal 142b representing a varying electrical conductance of the second sensor layer 102 and to apply a plurality of time windows 201, ..., 205 to the second sensor layer conductance signal 142b, said time windows 201, ... 205 each having a window length corresponding to a period length of a single period 200 of the applied predetermined time-continuous periodic temperature profile 140.

The second circuitry may further be configured to apply a Discrete-Fourier-Transformation (DFT) to one or more of said plurality of time windows 201, ..., 205 for obtaining, for each of said one or more time windows 201, ..., 205, discrete frequency spectrum data 144 of the DFT-transformed second sensor layer conductance signal 142b representing a current gas concentration of the analyte gas.

Due to the differently modified active material 111, the second sensor layer conductance signal 142b of the second sensor layer 102 differs from the sensor layer conductance signal 142b of the first sensor layer 101 in case an identical concentration of the analyte gas is present at the sensor surface 110.

Additionally or alternatively, the second sensor layer 102 may not only be able to determine a different concentration of the analyte gas, but the second sensor layer 102 may also be configured to determine a different type of analyte gas. For example, the first sensor layer 101 may be sensitive to NO₂, while the second sensor layer 102 may be sensitive to O₃.

According to such an embodiment, the sensor surface 110 may comprise at least a second chemically sensitive sensor layer 102 comprising an active material 111 for adsorbing and desorbing gas molecules 133 of another analyte gas, i.e. of a different type of analyte gas compared to the first sensor layer 101. For instance, the second chemically sensitive sensor layer 102 may comprise a differently modified active material 111 than the first sensor layer 101.

In this case, the second circuitry may again be configured to determine a second electrical sensor layer conductance signal 142b representing a varying electrical conductance of the second sensor layer 102 and to apply a plurality of time windows 201, ..., 205 to the second sensor layer conductance signal 142b, said time windows 201, ..., 205 each having a window length corresponding to a period length of a single period 200 of the applied predetermined time-continuous periodic temperature profile 140.

The second circuitry may further be configured to apply a Discrete-Fourier-Transformation (DFT) to one or more of said plurality of time windows 201, ..., 205 for obtaining, for each of said one or more time windows 201, ..., 205, discrete frequency spectrum data 144 of the DFT-transformed second sensor layer conductance signal representing a current gas concentration of the another analyte gas.

Also with respect to the second sensor layer 102, the second circuitry may be configured to apply at least one of a Discrete Fourier Transformation (DFT), a Fast Fourier Transformation (FFT), a Short-Time Fourier Transformation (STFT) or a Goertzel-Algorithm to said one or more time windows 201, ..., 205 for obtaining the discrete frequency spectrum data 144.

Up to now, the innovative concept was discussed with reference to devices and structures. However, the herein described innovative concept also concerns a corresponding method for operating a temperature-regulated chemo-resistive gas sensor 100 as discussed above.

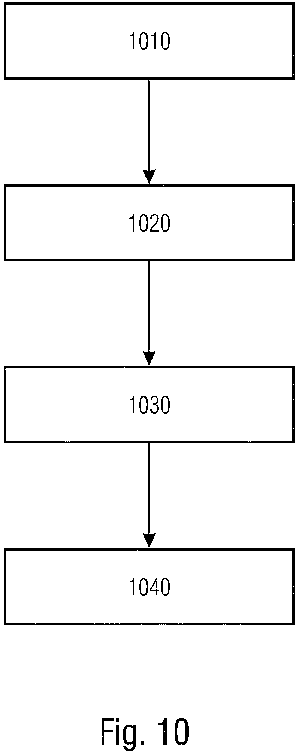

Figure 10 shows a block diagram of a corresponding method for operating a temperature-regulated chemo-resistive gas sensor 100 comprising a MEMS device comprising a heater 120 and a sensor surface 110 thermally coupled to the heater 120, the sensor surface 110 comprising at least one chemically sensitive sensor layer 101 comprising an active material 111 for adsorbing and desorbing gas molecules 133 of an analyte gas.

In block 1010 a predetermined time-continuous periodic temperature profile 140 is applied to the heater 120 for periodically heating the sensor surface 110, wherein an electrical conductance of the sensor layer 101 varies in response to the applied predetermined time-continuous periodic temperature profile 140.

In block 1020 an electrical sensor layer conductance signal 142a, 142b is determined, which represents the varying electrical conductance of the sensor layer 101 and a plurality of time windows 201, ..., 205 is applied to the sensor layer conductance signal 142a, 142b, wherein each of said applied time windows 201, ..., 205 has a window length corresponding to a period length of a single period 200 of the applied predetermined time-continuous periodic temperature profile 140.

In block 1030 discrete frequency spectrum data (144) of the sensor layer conductance signal is obtained for one or more of said plurality of time windows 201, ..., 205, the discrete frequency spectrum data 144 comprising at least one of the fundamental frequency and the second harmonic of the sensor layer conductance signal.

In block 1040 a current gas concentration of the analyte gas is determined based on the obtained discrete frequency spectrum data.

As mentioned above, the step of obtaining the discrete frequency spectrum data 144 may comprise a step of applying at least one of a DFT, FFT, STFT or a Goertzel-Algorithm to the one or more time windows 201, ..., 205.

According to an embodiment, the method may further comprise, prior to obtaining the discrete frequency spectrum data 144 (e.g. by applying a Discrete Fourier Transformation), a step of determining, for at least one of the applied plurality of time windows 201, ..., 205, a mean slope 902 of the sensor layer conductance signal 142b contained inside said at least one time window 201, ..., 205, and a step of using the determined mean slope 902 for flattening the sensor layer conductance signal 142b contained inside said at least one time window 201, ..., 205.

According to a further embodiment, the method may further comprise, prior to obtaining the discrete frequency spectrum data 144 (e.g. by applying a Discrete Fourier Transformation), a step of determining, for at least one of the applied plurality of time windows 201, ..., 205, a normalization factor, and a step of applying said normalization factor to the sensor layer conductance signal 142b contained inside said at least one time window 201, ..., 205.

According to a further embodiment, the method may further comprise a step of determining, for at least one of the applied plurality of time windows 201, ..., 205, a gas-specific phase angle Δϕ between the discrete frequency spectrum data 144 of the (e.g. DFT-transformed) sensor layer conductance signal contained inside said at least one time window 201, ..., 205 and the periodic temperature profile 140 applied during the window length of said at least one time window 201, ..., 205, and a step of determining the current gas concentration based on said gas-specific phase angle Δϕ.

Summarizing, it is suggested to apply a predetermined time-continuous and periodic temperature profile for periodically and time-continuously heating the sensor between a first temperature range, at which molecules of an analyte gas are adsorbed, and a second higher temperature range, at which adsorbed molecules are desorbed. The conductance of the sensor may be measured, wherein the conductance follows the temperature profile, i.e. the conductance is represented by a conductance signal that is also periodical and time-continuous. Depending on the type and/or concentration of the present analyte gas, the conductance signal may be distorted in a certain way. In a further step, the conductance signal may be analyzed so as to determine the frequency components that contribute to the distortion. According to the present innovative concept, a plurality of time windows may be applied to the conductance signal and, for each of the time windows, discrete frequency spectrum data of the sensor layer conductance signal may be determined, the discrete frequency spectrum data comprising at least one of the fundamental frequency and the second harmonic of the sensor layer conductance signal. A current gas concentration of the analyte gas may be determined based on the discrete frequency spectrum data. This innovative concept provides for an oversampling of the conductance signal which allows for a significantly faster sensor response.

In other words, the herein described innovative principle concerns a signal processing technique ("oversampling" method) which increases the sensing rate of periodic (e.g. sine) temperature profile gas sensors 100.

The herein described "oversampling" method may be based on a dynamic sensing method, containing a periodic temperature signal 141, resistance or conductance measurement, respectively and signal processing with DFT (Discrete Fourier Transformation). Figure 6 shows additional "readout periods", which may be triggered inside each temperature modulation period 200. This can be done with a sliding time window. Figures 9A to 9E show an optional preprocessing of each period 911, 912, 913 before DFT. Each sine pulse 911, 912, 913 may be flattened and normalized. Figures 7A to 7C explain an auto-phasing of DFT results 144 with the temperature reference 145. In this non-limiting example, a DFT was also applied to the periodic temperature signal 141 resulting in the DFT component, vector 145. The phase angle ϕ of the temperature DFT component 145 gives the reference. With this reference the phase shift of the additional readout periods can be neglected. Figures 8A and 8B show a gas sensing example with an angle of the first DFT harmonics without (Figure 8A) and with (Figure 8B) the innovative method.

The herein described innovative principle further suggests to provide a temperature-regulated chemo-resistive gas sensor 100 comprising a MEMS device and an integrated circuitry, e.g. the above mentioned first and second circuitries comprising an integrated temperature regulation and data analyzing function. The MEMS device may comprise an integrated heater 120 and a sensor surface 110 comprising one or more chemically sensitive sensor layers 101, 102, 103, 104.

Each of the sensor layers 101, 102, 103, 104 may comprise a different, or a differently modified active material 111. The active material 111 may comprise, or be made from, graphene. This can be pure graphene, or modified graphene. For example, graphene flakes may be mixed with functional nanoparticles or salts, wherein the latter influence the behavior of the otherwise pure graphene flakes. However, any other material being suitable of adsorbing and desorbing molecules 133 of an analyte gas may be employed as the active material 111.

Graphene may be advantageous since it has no chemical reactions with the molecules 133 of the analyte gas at the sensor surface 110. The adsorption and/or desorption of the molecules 133 at the graphene surface will only be influenced by the regulated temperature modulation, wherein the number of adsorbed molecules 133 influences the electrical sensor conductance. This is a distinction over MOx-based gas sensors.

The first and second circuitry may be provided as one single integrated circuit, or as two separate circuits. In any case, the integrated temperature regulation, optionally feedback-controlled, may provide an exact periodic (e.g. sine) temperature profile 140, which is independent from environmental influences like temperature, pressure, humidity or airflow. Particularly, if graphene may be used as the active material 111, a feedback-controlled temperature regulation may be advantageous due to the relatively low operating temperatures of graphene. This is a further distinguishing feature over simply applying a periodic heating voltage without a feedback-controlled regulation.

The data analysis may be integrated fully or in part (preprocessing (DFT) and calculation of the gas concentration (possibly with Artificial Intelligence - Al) into the first and/or second circuitry.

So the herein described innovative principle may provide for some kind of Short-Time-FFT, wherein the applied window length exactly corresponds with a duration of exactly one period 200 of the temperature modulation. After each complete temperature period, a Short-Time-FFT will be applied to the sensor layer conductance signal 142b. Thus, for each period, a data set of the discrete frequency spectrum of the sensor layer conductance signal 142b may be obtained. The discrete frequencies correspond to the harmonics (integer multiples) of the temperature frequency. For calculating the gas concentration, the first three to four harmonics may be used. It was discovered that the most information is contained in the phase angle of the first harmonic (which is the basic frequency of the temperature modulation).

Without any adsorption or desorption of a sensor active analyte gas during the temperature modulation, the sensor layer conductance signal does not comprise any phase shift.

Thus, it is a surprising technical effect that the obtained angle value is entirely free from base drift. This is one of several big advantages of the present innovative concept because base line drift is a severe problem with chemo-resistive sensors, and in particular with graphene based sensors.

An integration of such gas sensors 100 according to the herein described innovative principle is one of the highly expected next steps in the evolution of mobile devices. This enables a real time gas sensing using a signal processing technique enabling an immediate gas prediction.

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Some or all of the method steps may be executed by (or using) a hardware apparatus, like for example, a microprocessor, a programmable computer or an electronic circuit. In some embodiments, one or more of the most important method steps may be executed by such an apparatus.

Depending on certain implementation requirements, embodiments of the present innovative concept can be implemented in hardware or in software or at least partially in hardware or at least partially in software. The implementation can be performed using a digital storage medium, for example a floppy disk, a DVD, a Blu-Ray, a CD, a ROM, a PROM, an EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the present innovative concept comprise a data carrier having electronically readable control signals, which are capable of cooperating with a programmable computer system, such that one of the methods described herein is performed.

Generally, embodiments of the present innovative concept can be implemented as a computer program product with a program code, the program code being operative for performing one of the methods when the computer program product runs on a computer. The program code may for example be stored on a machine readable carrier.

Other embodiments comprise the computer program for performing one of the methods described herein, stored on a machine readable carrier.

In other words, an embodiment of the inventive method is, therefore, a computer program having a program code for performing one of the methods described herein, when the computer program runs on a computer.

A further embodiment of the inventive methods is, therefore, a data carrier (or a digital storage medium, or a computer-readable medium) comprising, recorded thereon, the computer program for performing one of the methods described herein. The data carrier, the digital storage medium or the recorded medium are typically tangible and/or non-transitory.

A further embodiment of the inventive method is, therefore, a data stream or a sequence of signals representing the computer program for performing one of the methods described herein. The data stream or the sequence of signals may for example be configured to be transferred via a data communication connection, for example via the Internet.

A further embodiment comprises a processing means, for example a computer, or a programmable logic device, configured to or adapted to perform one of the methods described herein.

A further embodiment comprises a computer having installed thereon the computer program for performing one of the methods described herein.

A further embodiment according to the present innovative concept comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

The apparatus described herein may be implemented using a hardware apparatus, or using a computer, or using a combination of a hardware apparatus and a computer.

The methods described herein may be performed using a hardware apparatus, or using a computer, or using a combination of a hardware apparatus and a computer.

While this disclosure has been described with reference to illustrative embodiments, this description is not intended to be construed in a limiting sense. Various modifications and combinations of the illustrative embodiments, as well as other embodiments of this disclosure, will be apparent to persons skilled in the art upon reference to the description. It is therefore intended that the appended claims encompass any such modifications or embodiments.

## Claims

1. A temperature-regulated chemi-resistive gas sensor (100) comprising
a MEMS device (MEMS: Micro Electro Mechanical System) comprising a heater (120) and a sensor surface (110) thermally coupled to the heater (120), the sensor surface (110) comprising at least one chemically sensitive sensor layer (101) comprising an active material (111) for adsorbing and desorbing gas molecules (133) of an analyte gas,
a first circuitry configured to apply a predetermined time-continuous periodic temperature profile (140) to the heater (120) for periodically heating the sensor surface (110), wherein an electrical conductance of the sensor layer (101) varies in response to the applied predetermined time-continuous periodic temperature profile (140), and
a second circuitry configured to determine an electrical sensor layer conductance signal (142a, 142b) representing the varying electrical conductance of the sensor layer (101) and to apply a plurality of time windows (201, ..., 205) to the sensor layer conductance signal (142a, 142b), said time windows (201, ..., 205) each having a window length corresponding to a period length of a single period (200) of the applied predetermined time-continuous periodic temperature profile (140),
wherein the second circuitry is further configured to obtain for one or more of said plurality of time windows (201, ..., 205) discrete frequency spectrum data (144) comprising at least one of the fundamental frequency and the second harmonic of the sensor layer conductance signal, and to determine a current gas concentration of the analyte gas based on the obtained discrete frequency spectrum data (144).

2. The chemi-resistive gas sensor according to claim 1,
wherein the second circuitry is configured to apply at least one of
• a Discrete Fourier Transformation (DFT),
• a Fast Fourier Transformation (FFT), or
• a Goertzel-Algorithm
to said one or more time windows (201, ..., 205) for obtaining the discrete frequency spectrum data (144).

3. The temperature-regulated chemi-resistive gas sensor (100) according to claim 1 or 2,
wherein the second circuitry is configured to start the plurality of time windows (201, ..., 205) during a period length of one single period (200) of the predetermined time-continuous periodic temperature profile (140).

4. The temperature-regulated chemi-resistive gas sensor (100) according to any one of claims 1 to 3,
wherein the first circuitry comprises a feedback-controlled temperature regulation for controlling the applied predetermined time-continuous periodic temperature profile (140) such that,
during the period length of one single period (200) of the periodic temperature profile (140), the predetermined time-continuous periodic temperature profile (140) passes a first target temperature range (411) at which the sensor layer (101) adsorbs gas molecules (133) of the analyte gas and a second target temperature range (412) at which the sensor layer (101) desorbs adsorbed gas molecules (133) of the analyte gas.

5. The temperature-regulated chemi-resistive gas sensor (100) according to any one of claims 1 to 4,
wherein the applied predetermined time-continuous periodic temperature profile (140) has a time-continuous temperature increase (401) and a time-continuous temperature decrease (402) during the period length of one single period (200).

6. The temperature-regulated chemi-resistive gas sensor (100) according to any one of claims 1 to 5,
wherein the temperature profile (140) is represented by a temperature profile signal (141),
wherein said temperature profile signal (141) is sinusoidal.

7. The temperature-regulated chemi-resistive gas sensor (100) according to any one of claims 1 to 6,
wherein the second circuitry is configured to, prior to obtaining the discrete frequency spectrum data (144),
determine, for at least one of the applied plurality of time windows (201, ..., 205), a mean slope (902) of the sensor layer conductance signal (142b) contained inside said at least one time window (201, ..., 205), and
to use the determined mean slope (902) for flattening the sensor layer conductance signal (142b) contained inside said at least one time window (201, ..., 205).

8. The temperature-regulated chemi-resistive gas sensor (100) according to any one of claims 1 to 7,
wherein the second circuitry is configured to, prior to obtaining the discrete frequency spectrum data (144),
determine, for at least one of the applied plurality of time windows (201, ..., 205), a normalization factor, and
apply said normalization factor to the sensor layer conductance signal (142b) contained inside said at least one time window (201, ..., 205).

9. The temperature-regulated chemi-resistive gas sensor (100) according to any one of claims 1 to 8,
wherein the second circuitry is configured to
determine, for at least one of the applied plurality of time windows (201, ..., 205), a gas-specific phase angle (Δϕ) between
the discrete frequency spectrum data (144) of the sensor layer conductance signal belonging to said at least one time window (201, ... ,205)
and the temperature profile (140) applied during the window length of said at least one time window (201, ..., 205), and
determine the current gas concentration of the analyte gas based on said gas-specific phase angle (Δϕ).

10. The temperature-regulated chemi-resistive gas sensor (100) according to claim 9,
wherein the second circuitry is configured to
apply a Discrete Fourier Transformation to the temperature profile (140) that is applied during the window length of said at least one time window (201, ..., 205) in order to obtain a discrete Fourier-transformed temperature profile signal (145) for said at least one time window (201, ..., 205), and
determine, for said at least one time window (201, ..., 205), the gas-specific phase angle (Δϕ) between the discrete frequency spectrum data (144) of the sensor conductance signal and the discrete Fourier-transformed temperature profile signal (145).

11. The temperature-regulated chemi-resistive gas sensor (100) according to any one of claims 1 to 10,
wherein the discrete frequency spectrum data (144) of the sensor layer conductance signal correspond to harmonics of the frequency of the applied temperature profile (140), and
wherein the second circuitry is configured to determine the current gas concentration of the analyte gas based on at least the first harmonic, or based on the first harmonic only.

12. The temperature-regulated chemi-resistive gas sensor (100) according to any one of claims 1 to 11,
wherein the active material (111) of the at least one chemically sensitive sensor layer (101) comprises a conducting or a semi-conducting material having an electrical conductivity that is variable based on the type and/or number of gas molecules that are adsorbed at the surface of said material.

13. The temperature-regulated chemi-resistive gas sensor (100) according to claim 12,
wherein the active material (111) of the at least one chemically sensitive sensor layer (101) comprises at least one of
• a graphene-based material,
• modified graphene,
• a mono-crystalline graphene monolayer, or
• a 2D material.

14. The temperature-regulated chemi-resistive gas sensor (100) according to any one of claims 1 to 13,
wherein the sensor surface (110) comprises at least a second chemically sensitive sensor layer (102) comprising an active material (111) for adsorbing and desorbing gas molecules (133) of the analyte gas, wherein said second chemically sensitive sensor layer (102) comprises a differently modified active material (111) than the at least one chemically sensitive sensor layer (101),
wherein the second circuitry is configured to determine a second electrical sensor layer conductance signal (142b) representing a varying electrical conductance of the second sensor layer (102) and to apply a plurality of time windows (201, ..., 205) to the second sensor layer conductance signal (142b), said time windows (201, ..., 205) each having a window length corresponding to a period length of a single period (200) of the applied predetermined time-continuous periodic temperature profile (140),
wherein the second circuitry is further configured to obtain for one or more of said plurality of time windows (201, ..., 205) discrete frequency spectrum data (144) comprising at least one of the fundamental frequency and the second harmonic of the second sensor layer conductance signal (142b), and to determine a current gas concentration of the analyte gas based on the obtained discrete frequency spectrum data (144) of the second senor layer conductance signal (142b),
wherein, due to the differently modified active materials (111), the second sensor layer conductance signal (142b) of the second sensor layer (102) differs from the sensor layer conductance signal (142b) of the at least one sensor layer (101) in case an identical concentration of the analyte gas is present at the sensor surface (110).

15. The temperature-regulated chemi-resistive gas sensor (100) according to any one of claims 1 to 13,
wherein the sensor surface (110) comprises at least a second chemically sensitive sensor layer (102) comprising an active material (111) for adsorbing and desorbing gas molecules (133) of another analyte gas, wherein said second chemically sensitive sensor layer (102) comprises a differently modified active material (111) than the at least one chemically sensitive sensor layer (101),
wherein the second circuitry is configured to determine a second electrical sensor layer conductance signal (142b) representing a varying electrical conductance of the second sensor layer (102) and to apply a plurality of time windows (201, ..., 205) to the second sensor layer conductance signal (142b), said time windows (201, ..., 205) each having a window length corresponding to a period length of a single period (200) of the applied predetermined time-continuous periodic temperature profile (140),
wherein the second circuitry is further configured to obtain for one or more of said plurality of time windows (201, ..., 205) discrete frequency spectrum data (144) comprising at least one of the fundamental frequency and the second harmonic of the second sensor layer conductance signal, and to determine a current gas concentration of the another analyte gas based on the obtained discrete frequency spectrum data (144) of the second senor layer conductance signal (142b),
wherein the another analyte gas being detectable by means of the second chemically sensitive sensor layer (102) is of a different kind than the analyte gas being detectable by means of the at least one chemically sensitive sensor layer (101).

16. A method for operating a temperature-regulated chemi-resistive gas sensor (100) comprising a MEMS device comprising a heater (120) and a sensor surface (110) thermally coupled to the heater (120), the sensor surface (110) comprising at least one chemically sensitive sensor layer (101) comprising an active material (111) for adsorbing and desorbing gas molecules (133) of an analyte gas, wherein the method comprises at least the following steps:
applying a predetermined time-continuous periodic temperature profile (140) to the heater (120) for periodically heating the sensor surface (110), wherein an electrical conductance of the sensor layer (101) varies in response to the applied predetermined time-continuous periodic temperature profile (140),
determining an electrical sensor layer conductance signal (142a, 142b) representing the varying electrical conductance of the sensor layer (101) and applying a plurality of time windows (201, ..., 205) to the sensor layer conductance signal (142a, 142b), said time windows (201, ..., 205) each having a window length corresponding to a period length of a single period (200) of the applied predetermined time-continuous periodic temperature profile (140), and
obtaining, for one or more of said plurality of time windows (201, ..., 205), discrete frequency spectrum data (144) comprising at least one of the fundamental frequency and the second harmonic of the sensor layer conductance signal, and to determine a current gas concentration of the analyte gas based on the obtained discrete frequency spectrum data (144).

17. A computer-readable storage medium having a computer program stored thereon, for performing, when being executed on a computer or signal processor, the method of claim 16.
